Europäisches Patentamt

**European Patent Office**

Office européen des brevets

(19)

(11) Publication number: **0 199 586**
A2

(12)

# EUROPEAN PATENT APPLICATION

(21) Application number: **86303041.7**

(22) Date of filing: **22.04.86**

(51) Int. Cl.⁴: **C 07 K 3/02**
A 61 K 39/00, C 12 P 21/00
C 12 N 15/00, G 01 N 33/577
A 61 K 49/00, A 61 K 39/395

(30) Priority: **22.04.85 US 726201**

(43) Date of publication of application:
**29.10.86 Bulletin 86/44**

(84) Designated Contracting States:
**AT BE CH DE FR GB IT LI LU NL SE**

(71) Applicant: **HYBRITECH INCORPORATED**
**11095 Torreyana Road**
**San Diego California 92126(US)**

(72) Inventor: **Burnett, Karen**
**3814 Martha Street**
**San Diego California(US)**

(72) Inventor: **Leung, Julia**
**7924 Camino Jonata**
**San Diego California(US)**

(72) Inventor: **Oh, Esther**
**11117 Pegasus Avenue**
**San Diego California(US)**

(74) Representative: **Goldin, Douglas Michael**
**J.A. KEMP & CO. 14, South Square Gray's Inn**
**London WC1R 5EU(GB)**

(54) **Tumour-associated antigen.**

(57) A novel tumour-associated antigen expressed by colon carcinoma is disclosed. The antigen, characterized by monoclonal antibody YCR010, is an O-linked glycoprotein having a molecular weight in the range of about 36,000 to about 39,000 and an isoelectric point within the range of about 5.1 to about 5.4. Antibodies directed against the antigen, methods for their production and diagnostic and therapeutic uses therefore are provided.

EP 0 199 586 A2

Croydon Printing Company Ltd

TITLE MODIFIED
see front page

- 1 -

## A NOVEL TUMOUR-ASSOCIATED ANTIGEN

The present invention relates to the characterization of antigens, particularly tumor-associated antigens. In another aspect, it relates to antibodies having specific reactivity with such antigens. In yet another aspect, it relates to methods for producing such antibodies as well as diagnostic and therapeutic uses therefor.

The potential role of monoclonal antibodies in the diagnosis and treatment of cancer in humans has been the focus of much recent investigation and speculation. Of particular interest are their use in immunoassays to monitor the course of disease, e.g., during therapy. Also, of particular interest are the potential applications of monoclonal antibodies for tumor imaging and therapy due to their capacity to bind to tumor-associated antigens in vivo.

Developments in monoclonal antibody technology have also made it possible to investigate the antigenic complexity of human tumors. Specifically, the precise immunoreactivity of monoclonal antibodies permits the identification and differentiation of cell surface antigens expressed by human tumors. The characterization, therefore, of such distinct tumor-associated antigens provides a means to more fully exploit the use of monoclonal antibodies for cancer diagnosis and therapy and to maximize the advances of monoclonal antibody technology.

To date, only a limited number of tumor-associated antigens are well characterized. Additionally, certain tumor-associated antigens useful as diagnostic or prognostic markers may not be present in all patients or during all stages and manifestations of the disease. Accordingly, there exists a need for further identification and characterization of unique tumor-associated antigens.

- 3 -

The present invention is predicated upon the discovery and characterization of a novel antigen present in human tissue, particularly normal colon and colon carcinoma. Accordingly, the invention is directed to a distinct tumor-associated antigen, characterized by its reactivity with monoclonal antibody YCR010.

In accordance with the present invention, antibodies having specificity for the antigen defined herein and methods for the production of such antibodies are provided. Additionally, the invention is directed to the use of such antibodies for the in vitro detection and diagnosis of cancer by immunohistochemical and immunoassay methods. The invention is further directed to the use of such antibodies for the in vivo diagnosis and treatment of cancer in humans.

As indicated above, the present invention provides a novel tumor-associated antigen. In accordance with the invention, monoclonal antibody YCR010, generated by hybrid cell line ATCC Deposit #HB8787, is utilized for characterization of this previously undescribed antigen.

The physiochemical and immunological properties of the antigen of the present invention, and particularly, its reactivity with monoclonal antibody YCR010, permit its characterization and differentiation from other antigens present in normal tissue and tumor tissue. Accordingly, the

- 4 -

identifying characteristics and properties of the antigen provided by the present invention are as follows:

a) The antigen is a membrane component of normal colon tissue and the mucosa of colon carcinoma. Standard ELISA procedures (enzyme-linked immunoabsorbent binding assay) using monoclonal antibody YCR010 indicate the presence of the antigen in plasma membranes purified from human colon tissue and the mucosa of colon carcinoma. By comparison, YCR010 exhibits no appreciable reactivity with membranes purified from such other benign tissues as liver, kidney, prostate, bladder, brain and pancreas.

b) Standard immunohistochemical procedures demonstrate the presence of the antigen in benign colon and colon carcinoma as a strong reactivity with YCR010 is shown by immunoperoxidase staining of such tissue. A weak reactivity or no reactivity is shown with other benign tissues, including prostate, lung, liver, kidney, pituitary, skin and breast.

c) SDS - polyacrylamide gel electrophoresis (SDS-PAGE) and Western immunoblot analysis reveal that the antigen has a molecular weight in the range of about 36,000 to about 39,000. Modification of the antigen by treatment with proteinase K and by treatment with sodium periodate prior to SDS-PAGE and Western immunoblot analysis destroys the antigen's reactivity with YCR010, indicating the antigen is glycoprotein in nature.

d) The antigen is an O-linked glycoprotein. Mild alkali treatment of the antigen and treatment of the antigen with N-glycanase confirms the glycoprotein nature of the antigen and demonstrates that the antigen is an O-linked glycoprotein.

e) Isoelectric focusing of the antigen using a standard flat-bed isoelectric focusing technique indicates an isoelectric point within the range of about 5.1 to about 5.4.

Summarizing the foregoing, the antigen of the present invention, which may be derived from a source selected from normal human tissue or human tumor tissue, is characterized as an O-linked glycoprotein having a molecular weight within the range of about 36,000 to about 39,000 and having an isoelectric point within the range of about 5.1 to about 5.4. Further, the tumor-associated glycoprotein of the present invention is expressed by normal colon and colon carcinoma.

Of particular importance in distinguishing the antigen of the present invention from other antigens, including other tumor-associated antigens, is the specificity of monoclonal antibody YCR010 for at least one determinant of the antigen characterized herein. Additionally, the specific reactivity of YCR010 for the antigen defined by the invention provides a means for isolation and purification of the antigen from other material of human origin, and ultimately

0199586

- 6 -

characterization of antigenic determinants. Such a purified antigen and determinants thereof are useful in the production of monoclonal and polyclonal antibodies for diagnostic and therapeutic application using techniques well known in the art. For example, murine hybridomas producing monoclonal antibodies may be obtained. In other cases, the antigen may be used to stimulate an immune response in a rabbit, goat, or other animal from whose serum polyclonal antibodies may be obtained. In addition, the antigen may be used for the characterization of antibodies of interest, e.g., monoclonal antibodies, or antibodies present in human tissue, blood, or other body fluids.

In accordance with the present invention, monoclonal antibodies having specificity for the antigen characterized herein, and methods for their production, are provided. Preferably, such monoclonal antibodies are of the IgG class and possess an immunoreactivity with the tumour-associated glycoprotein of the present invention substantially similar to that of monoclonal antibody YCR010. Such monoclonal antibodies are useful in the detection, diagnosis, and treatment of cancer in humans, particularly colorectal carcinoma. Further, such antibodies have application in the further isolation and purification of the antigen provided by the invention and the characterization of precise determinants.

Monoclonal antibodies as described above may be produced generally following the method of Kohler and Milstein, Nature, 256, 495 - 497 (1975). In accordance with the present invention, a mouse or other suitable host is immunized with the purified antigen of the invention or a membrane fraction of human tumour tissue derived from a colon carcinoma. Following immunization, tne spleen cells of tne immunized mouse are fused with the cells from a suitable mouse myeloma line to obtain a mixture of hybrid cell lines. The hybrid cell lines are cultured in a suitable medium, and thereafter, those hybrid cell lines producing an antibody having specific reactivity with the antigen characterized herein are selected and cloned, and the antibody produced is recovered.

The present invention suggests methods for the in vitro detection and diagnosis of cancer in humans, especially colorectal carcinoma. Characterization of the unique tumor-associated glyprotein of the invention as set forth herein permits its detection in patient tissue samples by immunohistochemical methods and/or in patient fluid samples by in vitro immunoassay methods. A determination of the presence and/or quantity of the tumor-associated antigen of the present invention in patient specimens by immunohistochemical and/or immunoassay procedures is of diagnostic utility and may be indicative of, or correlate with, the progression of the disease state.

Immunohistochemical methods for the detection of antigens in patient tissue specimens are well known to the art and need not be described in detail. Briefly, in the context of the present invention, a tissue specimen obtained from a suspected cancer patient is contacted with an antibody, preferably a monoclonal antibody, having specificity for the tumor-associated glycoprotein characterized herein. Particularly preferred for use is monoclonal antibody YCR010. The sites at which the antibody is bound is thereafter determined by selective staining of the tissue specimen by immunohistochemical procedures.

Similarly, methods for the in vitro detection of antigenic substances in patient fluid samples by immunoassay procedures are well known to the art and require no repetition. For purposes of the present invention, a patient fluid sample is contacted with at least one antibody, preferably a monoclonal antibody, having specific reactivity with the tumor-associated glycoprotein of the invention and the antibody bound to sample components is determined. Qualitative and/or quantitative determinations of the presence of the antigen defined by the invention may be accomplished by competitive or non-competitive immunoassay procedures. Monoclonal antibodies or polyclonal antibodies may be suitably utilized provided such antibodies possess the requisite specificity for the antigen provided by the present invention. Preferably, monoclonal antibody YCR010 is utilized. Additionally, the immunoassays preferred for use are two-site immunometric assays employing monoclonal antibodies which are selected to bind to non-interfering determinants of the antigen characterized herein.

The present invention further suggests methods for the in vivo diagnosis and therapy of cancer, particularly colorectal carcinoma. Methods for tumor localization and detection may be performed, in accordance with the present invention, by administering to a suspected cancer patient a predetermined effective amount of an antibody having specific reactivity with the tumor-associated glycoprotein of the present invention and detecting the sites of localization of the antibody by standard imaging techniques. The antibody, preferably YCR010, is administered to the patient in a pharmaceutically acceptable carrier and labeled, preferably with a radionuclide emitting gamma-radiation, so as to permit detection.

In accordance with methods permitted by the present invention for cancer therapy, a predetermined effective amount of an antibody, preferably a monoclonal antibody, having specificity for the tumor-associated antigen characterized by the invention is administered to a cancer patient. The antibody, preferably YCR010, is administered to the cancer patient in a pharmaceutically acceptable carrier and conjugated with a suitable therapeutic agent, e.g., radioisotopes, preferably emitters of beta particles, drugs, toxins or biological proteins, selected for delivery to the tumor site.

Additionally, in the context of _in vivo_ cancer diagnosis and therapy, those skilled in the art will appreciate that antibody preparations comprising mixtures of antibodies or fragments thereof having specificity for the tumor-associated antigen of the invention may be used in certain instances to enhance the detection, localization and treatment of tumors.

The present invention may be better understood by reference to the following non-limiting example.

## EXAMPLE I

### Production of Monoclonal Antibody YCR010

Lymph node lymphocytes obtained from a patient with colon carcinoma were thawed and cultured overnight in RPM1-1640 (Flow Laboratories, Alexandria, VA) containing 10% fetal bovine serum (PBS). $30 \times 10^6$ lymphocytes and $30 \times 10^6$ of P3/HT, (a subline of P3x63Ag8.653) were fused with 35% polyethylene glycol-1000 in serum-free RPMI-7.5% dimethyl sulfoxide. Cells were plated out at densities of $10^5$ total cells per well in 96-well plates (Costar, Cambridge, MA) in RPMI-10% FBS - HAT ($10^{-4}M_w$ hypoxanthine, $4 \times 10^{-7}m$ aminopterin and $1.6 \times 10^{-5}M$ thymidine). Cultures were maintained for 2 weeks in 5% $CO_2$ and thereafter supernatant was replaced with fresh HAT media once per week. Supernatant from wells with growing cultures were sampled at day 40 and tested for the presence of Ig by enzyme-linked immunoabsorbent binding assay (ELISA). Ig-producing clones were then screened by ELISA for specific reactivity with membranes and cytosols from colon tumors. The monoclonal antibody designated as YCR010 was characterized further and selected for use in the characterization of the antigen as provided herein.

## EXAMPLE II

### Antigen Characterization

Monoclonal antibody YCR010 was used to characterize the antigen of the present invention by the procedures set forth herein.

Membrane and cytosol fractions of human tissue were utilized for antigen characterization, as well as for screening the reactivity of monoclonal antibody YCR010 and prepared as follows:

Surgical and autopsy specimens collected within ten hours after death were cut into blocks and stored at -80°C. Tissue was homogenized in four volumes of 10mM tris-HCl pH7.5, 2mM calcium chloride, 2mM phenylmethylsulfonate (homogenization buffer) at 4°C in a Dounce homogenizer. All subsequent steps were carried out at 4°. The homogenate was centrifuged at 100xg for 5 minutes to remove nuclei and intact cells. The supernatant was removed and centrifuged at 130,000xg for one hour. The supernatant from this high speed centrifugation was removed and designated as the cytosol fraction. The pellet was resuspended in one volume of homogenization buffer and layered on a 40%/20% discontinuous sucrose gradient in 10mM tris-HCl pH7.2. The gradient was centrifuged at 130,000xg for 17 hours. Material at the 40%/20% interphase was pipetted off, and diluted 5-fold in homogenization buffer and centrifuged for 60 minutes at 25,000xg. The pellet was resuspended in homogenization buffer, aliquoted and stored at 80°C.

## Membrane/Cytosol ELISA

The presence of antigen reactive with monoclonal antibody YCR101 in purified membrane and cytosol fractions of normal human tissue and human tumor tissue was determined by an enzyme-linked immunoabsorbent binding assay (ELISA) as measured by absorbance at 490 nm.

To perform the membrane/cytosol ELISA, 10µg/well of cytosol or 2µg/well of membrane, prepared as described above, was dried onto flat-bottom microtiter plates (Dynatech, Alexandria, VA) at 37°C overnight. The plates were washed three times with cold tap water. 50ul of 20% horse serum in PBS (phosphate buffered saline, 0.15M NaCl, 20mM sodium phosphate pH7.2) was added followed by 50ul of hybridoma supernatant and incubated for one hour at room temperature. After washing, the wells were incubated with 100ul of a monoclonal mouse anti-human IgM-HRP conjugate (ZSAG11, conjugated by the method of Wilson & Nakane, 1978) diluted 1:1000 in 5% horse serum-PBS for one hour. Bound enzyme was detected by incubation with 100ul of 1ug/ml o-phenylenediamine, 0.03% $H_2O_2$ in 0.1M citrate-phosphate buffer pH5.0, for 30 minutes in the dark. Wells were quenched by the addition of 50ul per well of $NH_4 SO_4$. Absorbance at 490nm was read in a Dynatech ELISA plate reader.

As shown by Table I below, YCR010 was reactive with membrane fractions of mucin colon carcinoma and normal colon, indicating the presence of the antigen of the invention in mucin colon carcinoma and normal colon membranes. YCR010 was unreactive with membrane fractions of other tissues including normal lung, liver, prostate, bladder, brain and pancreas.

## Immunohistochemical Determination

The presence of antigen reactive with YCR010 in normal human tissue and human tumor tissue was determined by immunoperoxidase staining of human tissue.

Sections of frozen tissue blocks, obtained from surgical and autopsy specimens collected within ten hours after death and stored at -80°C were cut 4-6 micron sections on the microtome/cryostat. The sections, mounted on glass slides, were briefly air-dried and then dipped in acetone. An indirect immunoperoxidase assay, essentially as described by Taylor, Arch. Pathol. Lab. Med., 102, 13 (1978) , was used to stain these slides. Sections were rehydrated by incubating in phosphate-buffered saline (PBS) for 5 minutes. YCR010 antibody was overlayed onto the sections and incubated in a humid chamber for one hour. Antibody was washed off the sections with PBS and the sections were then immersed in PBS for 5 minutes. The sections were overlayed with a 1:50 dilution of peroxidase-conjugated goat anti-human IgM antibody (Tago Chemicals, Burlingame, CA) and incubated for 30 minutes in a humid chamber. Antibody was washed off with PBS. The color reaction was developed with 1mg/ml of diaminobenzidine and 0.03% $H_2O_2$. Slides were counterstained with hematoxylin easin.

Table II below indicates that YCR010 had a positive reactivity with normal colon and colon carcinoma, as measured by the intensity of the staining of tissue, and a weak or no reactivity with other tissues, including normal prostate, lung, liver, kidney, pituitary, skin and breast.

Biochemical Analysis of Antigen

The protein nature and molecular weight of the antigen characterized by YCR010 were determined by SDS polyacrylamide gel electrophoresis (SDS-PAGE), Western immunoblot analysis, and immunostaining. 25g of colon membrane proteins were dissolved in gel sample buffer (0.125M Tris pH6.8, 4% SDS, 20% glycerol, 0.002% bromophenol blue, 5% 2-mercaptoethanol) and separated by discontinuous SDS-PAGE (Laemmli, 1970) on a 7.5% or 10% gel with a 3% stacking gel.

The separated proteins were electrophoretically transferred to nitrocellulose (0.1um, Schleicher & Schuell) at 200mA for 3 hours according to the method of Towbin et al PNAS 76:43-50 (1979). The nitrocellulose replicas were then incubated with 3% bovine serum albumin (BSA)-PBS for 30 minutes, after which they were incubated with hybridoma supernatant diluted 1:1 with 5% skim milk-0.001% thimerosal-0.1% anti-foam A emulsion (Sigma Chemical Co., St. Louis, MO) overnight, in sealed pouches. The nitrocellulose strips were washed with PBS-0.1% Tween for 30 minutes with 5 changes of buffer, and incubated with 125[1]-labeled goat anti-human IgM (Tago, Burlingame, CA), 500,000cpm/ml skim milk reagent, for 2 hours. After washing with PBS-0.1% Tween, the nitrocellulose strips were air-dried and exposed on X-ray film (Kodak XAR-5) for 18-72 hours with intensifying screen (Cronex DuPont Lightning Plus) at -70°.

To further determine the nature of the antigen, membranes prepared as described above were subjected to the following treatments prior to SDS-PAGE and Western immunoblot analysis.

a)  Methanol:  the membrane suspension was incubated with 5 volumes of methanol for one hour at 4°, microfuged, and the precipitate washed twice with ethanol. The precipitate was dissovled in gel sample buffer for SDS-PAGE.

b)  Sodium periodate:  membranes were incubated in 20mM sodium periodate for one hour at 4°.

c)  Neuraminidase:  the membrane suspension was adjusted to pH5-6 with acetic acid and incubated for one hour at 37° with 10mU of neuraminidase (160 units/mg., Sigma Chemical Co., St. Louis, MO).

d)  Proteinase K:  the membrane suspension was incubated with 1.25mg/ml of proteinase K (20 units/mg., Boehringer-Mannheim) for one hour at 37°.

All reactions were stopped by adding gel sample buffer.

By SDS-PAGE and Western immunoblot analysis the molecular weight of the antigen was determined to be within the range of about 36,000 to about 39,000. Chemical and enzyme modification of the antigen followed by SDS-PAGE and and Western immunoblot analysis demonstrated the glycoprotein nature of the antigen. Specifically, reactivity of the antigen with YCR010 was shown to be destructible by treatment of the antigen with proteinase K as well as sodium periodate as described above. The loss of reactivity of the antigen with YCR010 did not result from the alternative modifications described above.

## Determination of O-Linked Glycoprotein

The nature of the antigen was further characterized to be an O-linked glycoprotein by mild alkali and N-glycanase treatment.

Mild Alkali Treatment:

Membranes were treated with 0.1N NaOH overnight at room temperature, dialyzed with PBS and airfuged at 100,000xg for 30 minutes. The pellet was dissolved in gel sample buffer for SDS-PAGE and western immunoblot. The supernatant was spotted and dried on nitrocellulose and immunostained as in Western blot analysis.

N-Glycanase treatment:

25μg membrane proteins was boiled in the presence of 0.5% SDS-0.1M B-mercaptoethanol, diluted with an equal volume of 0.2M sodium phosphate pH8.6-10mM EDTA-1% NP40 and incubated with 1 unit of N-glycanase (260 units/ml., Genzyme Corporation, Boston, MA) overnight at 30°. The sample was dissolved in gel sample buffer for SDS-PAGE and Western immunoblot analysis.

## Isoelectric Focusing

Isoelectric focusing of the antigen characterized by YCR010 was determined using a standard flat-bed isoelectric focusing technique on polyacrylamide gel as described below.

Membranes were extracted in 6M urea-0.5% NP40-0.05% SDS by sonication (Heat Systems, Ultrasonics, Inc., model W-375) and subjected to horizontal isoelectric focusing (Multiphor II, LKB) on PAGplates pH3.5-9.5 (LKB) for 1-1/4 hours at 5°, at the suggested power settings of 30W, 50mA and 1500V. After focusing the separated proteins were passively transferred to nitrocellulose in Tris-buffered saline (TBS, 0.02M Tris-0.15M NaCl pH8.0) overnight, immunostained and visualized as in Western blot analysis.

Isoelectric focusing of the antigen by this technique revealed its isoelectric point to be within the range of about 5.1 to about 5.4.

### TABLE I

Monoclonal Antibody YCR010 Reactivity
with Membranes and Cytosol Preparations

|                        | Membranes | Cytosols |
|------------------------|-----------|----------|
| Colon carcinoma        | 0.00      | 0.00     |
| Colon carcinoma        | 0.00      | 0.00     |
| Mucin colon carcinoma  | 0.61      | 0.00     |
| Benign colon           | 0.00      | 0.00     |
| Benign colon           | 1.06      | 0.00     |
| Benign colon           | 0.86      | 0.00     |
| Benign lung            | 0.00      | 0.00     |
| Benign lung            | 0.00      | 0.00     |
| Benign liver           | 0.00      | 0.34     |
| Benign kidney          | 0.00      | 0.13     |
| Benign prostate        | 0.00      | 0.00     |
| Benign bladder         | 0.00      | 0.00     |
| Benign brain           | 0.00      | 0.00     |
| Benign pancreas        | 0.00      | 0.00     |

## TABLE II

Immunohistological Reactivity of YCR010 on Human Tissues

| Tissue | # Positive/# Tested |
|---|---|
| Colon carcinoma | 4/8 |
| Benign colon | 2/4 |
| Benign prostate | 0/1 |
| Benign lung | 0/1 |
| Benign liver | 0/1 |
| Benign kidney | 0/1 |
| Benign pituitary | 0/1 |
| Benign skin | 0/1 |
| Benign breast | 1/1 (weak) |

- 20 -

CLAIMS

1. An antigen derived from a source selected from normal human tissue or human tumor tissue, said antigen being characterized as a glycoprotein having a molecular weight within the range of about 36,000 to about 39,000 and an isoelectric point within the range of about 5.1 to about 5.4.

2. The antigen of Claim 1 wherein said antigen is an O-linked glycoprotein.

3. The antigen of Claim 2 wherein said antigen is further characterized as having at least one antigenic determinant reactive with monoclonal antibody YCR010.

4. The antigen of Claim 3 wherein the reactivity of said antigen with monoclonal antibody YCR010 is destructible by treatment of said antigen with proteinase K or sodium periodate.

5. The antigen of Claim 4 wherein said antigen is a tumor-associated antigen expressed by colorectal carcinoma.

6. A method for producing monoclonal antibodies for use in the detection, diagnosis and treatment of cancer in humans comprising:

immunizing a mouse or other suitable host a purified antigen of Claims 1, 2, 3, 4 or 5 or a membrane fraction of human tumor tissue derived from a human colon carcinoma;

fusing spleen cells of said immunized mouse or other suitable host with suitable mouse myeloma cells, thereby obtaining a mixture of hybrid cell lines;

culturing said hybrid cell lines in a suitable medium;

selecting and cloning hybrid cell lines producing an antibody having specific reactivity with an antigen of Claims 1, 2, 3, 4 or 5; and recovering monoclonal antibody thus produced.

7. A monoclonal antibody prepared by the method of Claim 6.

8. The monoclonal antibody of Claim 7 wherein said antibody is an IgG antibody.

9. The monoclonal antibody of Claim 7 wherein said antibody is YCR010 generated by hybrid cell line ATCC Deposit #HB8787.

10. A monoclonal antibody of the IgM class having specific reactivity with a tumor-associated glycoprotein characterized as having a molecular weight within the range of about 36,000 to about 39,000, an isoelectric point within the range of about 5.1 to about 5.4, and a reactivity with said monoclonal antibody which is destructible by treatment of said glycoprotein with proteinase K or sodium periodate.

11. The monoclonal antibody of Claim 10 wherein said tumor-associated glycoprotein is expressed by colorectal carcinoma.

12. The monoclonal antibody of Claim 11 wherein said antibody is YCR010.

13. A method for the detection and diagnosis of cancer in a suspected cancer patient comprising contacting a tissue specimen obtained from said patient with an antibody having specific reactivity with the antigen of Claims 1, 2, 3, 4 or 5 and determining the sites on said specimen to which said antibody is bound by immunohistochemical means.

14. The method of Claim 13 wherein said antibody is a monoclonal antibody.

15. The method of Claim 14 wherein said antibody is YCR010.

16. A method for the in vitro detection and diagnosis of cancer in a suspected cancer patient comprising contacting a fluid sample obtained from said patient with at least one antibody having specific reactivity with the antigen of Claims 1, 2, 3, 4 or 5 and determining the binding of said antibody to components of said fluid sample by means of an immunoassay.

17. The method of Claim 16 wherein said antibody is a monoclonal antibody.

18. The method of Claim 17 wherein said antibody is YCR010.

19. The method of Claim 16 wherein said immunoassay is a two-site immunometric assay and said antibodies are monoclonal antibodies selected to bind to non-interfering determinants of said antigen.

20. A method for the _in vivo_ detection and diagnosis of cancer in a suspected cancer patient comprising administering a predetermined effective amount of an antibody having specific reactivity with the antigen of Claims 1, 2, 3, 4 or 5, said antibody being administered in a pharmaceutically acceptable carrier and labeled so as to permit detection, and detecting the sites of localization of said antibody.

21. The method of Claim 20 wherein said antibody is a monoclonal antibody.

22. The method of Claim 21 wherein said antibody is YCR010.

23. A method for the treatment of cancer in a cancer patient comprising administering a predetermined effective amount of an antibody having specific reactivity with the antigen of Claims 1, 2, 3, 4 or 5, said antibody being administered in a pharmaceutically acceptable carrier and conjugated with a suitable therapeutic agent.

24. The method of Claim 23 wherein said antibody is a monoclonal antibody.

25. The method of Claim 24 wherein said antibody is YCR010.

26. A monoclonal antibody against the antigen of Claims 1, 2, 3, 4 or 5.

27. The monoclonal antibody of Claim 26 wherein said antibody is a human monoclonal antibody.

28. The monoclonal antibody of Claim 26 wherein said antibody is a murine antibody.

29. Polyclonal antibodies against the antigen of Claims 1, 2, 3, 4 or 5.